# EUROPEAN PATENT SPECIFICATION

(11) **EP 3 895 684 B1**
(45) Date of publication and mention of the grant of the patent: **23.08.2023**
(21) Application number: 20169713.3
(22) Date of filing: 15.04.2020
(51) Int. Cl.: A61K 8/06, A61K 8/34, A61K 8/37, A61Q 19/10, A61Q 17/00

(54) **O/W EMULSION CONTAINING ETHANOL**
ÖL-IN-WASSER-EMULSION MIT ETHANOL
ÉMULSION H/E CONTENANT DE L'ÉTHANOL

(43) Date of publication of application: 20.10.2021
(73) Proprietor: Dr. Kurt Wolff GmbH & Co. KG, 33611 Bielefeld (DE)
(72) Inventor: SCHULZE ZUR WIESCHE, Erik, 33611 Bielefeld (DE); BÖPPLE, Petra, 33611 Bielefeld (DE); SCHEIDELER, Sabrina, 33611 Bielefeld (DE)
(74) Representative: Ter Meer Steinmeister & Partner

(56) References cited:
- EP-A1- 2 415 458
- US-A1- 2009 226 498
- DATABASE WPI Week 201771 Thomson Scientific, London, GB; AN 2017-58655W XP002800232, & CN 107 041 849 A (WINNER SUPPLIES MEDICAL CO LTD) 15 August 2017 (2017-08-15)

## Description

### Field of the invention

The present invention is directed to an O/W emulsion comprising 15.0 to 30.0 wt.-% ethanol and 1.0 to 10.0 wt.-% of cetearyl ethylhexanoate.

### Background of the invention

Disinfecting products of the prior art are commonly sold as solutions, ointments or gels containing more than 62 wt.-% ethanol. Gels are the most common sanitizing/disinfecting products. These gels have good disinfecting properties but can have skin-irritating or corrosive properties, in particular when applied multiple times a day (as is mandatory for health care professionals). Thus, the prior art disinfectants can leave the skin feeling irritated, rashed, dry and brittle.

Furthermore, such disinfectants can ultimately lead to unwanted side-effects for people with sensitive skin conditions, such as patients suffering from e.g. neurodermitis. The skin barrier may thus be weakened and applying disinfectant can have adverse effects. Instead of protecting the skin from external bacterial and viral pathogens, the skin barrier is weakened to such an extent that the use of a disinfectant may even promote infections eventually. Therefore, the disinfectants that are commercially available are unsuitable for people with skin sensitive conditions.

In addition, aqueous alcohol solutions and gels quickly evaporate on the skin, thus minimizing the contact-time of the ethanol with the skin and therefore leading to non-optimal disinfection. Even more, quick evaporation of the alcohol can lead to nausea and headaches when inhaled after prolonged or multiple exposure due to several applications per day.

Additionally, the prior art disinfectants containing ethanol are in particular unsuitable for people suffering from alcohol addiction and/or humans with decreased levels of the enzyme ADH (alcohol dehydrogenase) due to the quick evaporation and adsorption of the alcohol by inhalation.

Furthermore, the disinfectant compositions of the prior art are flammable having a flash point of around 17 °C. Therefore, special care needs to be taken during manufacturing, transport and storage of these disinfectants.

US 2009/0226498 A1 relates to a moisturizing hand sanitizer comprising water, an alcoholol and a high internal phase emulsion. EP 2 415 458 A1 relates to an oil-in-water emulsion composition comprising a specific silicone, ethanol, a thickening agent, and a polyol. CN 107 041 849 A relates to a medical sterilizing emulsion comprising 30-70 % ethanol.

In view of the above problems and disadvantages, it was an object of the present invention to provide an improved disinfecting composition that overcomes the insufficiencies of the prior art.

### Summary of the invention

These objects have surprisingly been solved by an oil-in-water emulsion of the present invention according to claim 1.

It has surprisingly been found according to the present invention that the oil-in-water emulsion both maintains high effectiveness against pathogens, in particular against bacterial infections and enveloped virus infections such as infections with SARS-CoV-2, while at the same time maintains the skin barrier function and is mild, stable, non-irritant, has moisturizing properties and provides a smooth, comfortable and clean feeling. A combined effect in terms of both high effectiveness against pathogens and improvement of skin barrier function was totally unexpected. In addition, it was surprisingly found that the composition leads to a prolonged contact of the skin with the ethanol and increased evaporation time of the ethanol.

In particular, it has surprisingly been found that according to the above aspect, the disinfecting properties can be maintained while at the same time the negative side effects of the available disinfecting compositions are decreased. In addition, it has surprisingly been found that the ethanol forms a stable and homogenous composition in the oil-in-water emulsion i.e. the formation of multiple distinct phases is not observed even after prolonged storage.

In another aspect, the present invention relates to the use of the oil-in-water emulsion of the present invention for use in the prevention of a bacterial, mycotic, or enveloped viral disease.

### Detailed description

The following definitions are relevant in connection with the embodiments of the present invention.

The meaning of the term "comprising" is to be interpreted as encompassing all the specifically mentioned features as well optional, additional, unspecified ones, whereas the term "consisting of" only includes those features as specified. Therefore, "comprising" includes as a limiting case the composition specified by "consisting of".

The term "weight-%" or "wt.-%" is to be understood to refer to the weight amount of the component relative to the total weight amount of the respective composition, if not specified otherwise.

Preferred embodiments according to the invention are defined hereinafter.

These embodiments are preferred, even if not denoted as such. The preferred embodiments are preferred alone or in combination. Further, it is to be understood that the following preferred embodiments refer to all aspects of the present invention, i.e. oil-in-water emulsion, the oil-in-water emulsion for use in the prevention of a bacterial or enveloped viral disease, and the non-therapeutic use.

In an embodiment, the invention relates to an oil-in-water emulsion comprising
(a) an oil phase;
(b) an aqueous phase; and
(c) an emulsifier,
wherein the emulsion further comprises 15.0 to 30.0 wt.-% ethanol and 1.0 to 10.0 wt.-% of cetearyl ethylhexanoate as a skin care component.

In a preferred embodiment, the emulsion comprises 15.0 to 25.0, 17.0 to 23.0 or 19.0 to 21.0 wt.-% ethanol.

The oil-in-water emulsion (in the following sometimes also referred to as O/W emulsion) comprises 1.0 to 10.0 wt.-% of cetearyl ethylhexanoate, preferably 2.0 to 8.0 wt.-% or 4.0 to 7.0 wt.-%, as a skin care component. It has surprisingly been found that in the presence of cetearyl ethylhexanoate, a composition can be provided that results in a smooth and comfortable feeling of the skin while at the same time providing a stable composition.

In an embodiment, the emulsifier is present in an amount of 1.0 to 15.0 wt.-%.

In an embodiment, the emulsifier is selected from the group of fatty alcohol esters, glycerol fatty acid esters, polyoxyethylene ethers of one or more fatty alcohols, polyglycerol ethers of fatty alcohols, polyglycerol esters of fatty acids, and mixtures thereof.

The term "fatty alcohol esters" refers to C₂-C₂₀ allkanoates of C₆-C₂₈ branched or linear alkyls or alkenyls, wherein the prefix "Cₓ-C_{y}" denotes the number of carbon atoms. It is preferred that the C₂-C₂₀ alkanoate is acetate. Non-limiting examples of fatty alcohol esters include n-octylacetate and lauryl acetate. These compounds can additionally act as fragrance in the oil-in-water composition.

In the present application the term "glycerol fatty acid ester" refers to a glycerol mono or di fatty acid ester, i.e. a compound composed of a molecule of glycerol linked to a single fatty acid via an ester bond. Examples are glycerol monostearate, glycerol monobehenate, glycerol monocaprylate, glycerol monocaprate and glycerol monolaurate.

Preferably, the polyoxyethylene ethers of one or more fatty alcohols is selected from the group of steareth-2, steareth-21, macrogol cetostearyl ether 12, ceteareth-25, macrogol cetostearyl ether 20 and mixtures of the aforementioned compounds. More preferably, the polyoxyethylene ethers of one or more fatty alcohols is selected from the group of ceteareth-25, macrogol cetostearyl ether 20 and mixtures of the aforementioned compounds. In the current composition ceteareth-25, macrogol cetostearyl ether 12 and macrogol cetostearyl ether 20 promote the dispersion of the oil phase in the aqueous of the emulsion. They have ideal dispersion properties due to the lengths of the respective alcohol residues.

The term "polyglycerol ethers of fatty alcohols" refers to a compound of the formula R¹O-(C₃H₆O₂)ₙ-H, wherein R¹ is a C₆-C₂₈ branched or linear alkyl or alkenyl group and n is an integer greater than 1, preferably an integer from 2 to 10. It is preferred that the oil-in water emulsion comprises 1.0 to 8.0 wt.-% of polyglycerol ethers of fatty alcohols, and/or 1.0 to 5.0 wt.-% of glycerol fatty acid esters, and/or 1.0 to 5.0 wt.-% of polyoxyethylene ethers of one or more fatty alcohols.

The term "polyglycerol esters of fatty acids" refers to compounds containing a polyglycerol unit as well as at least one C₆-C₂₆ alkyl or alkenyl carboxylic acid unit. These two units may be combined directly by an ester bond or contain a linking unit that links these two units together. Non-limiting example of this group are polyglyceryl-3 methylglucose distearate, polyglycerol ester of interesterified ricinoleic acid, polyglycerol polycrinoleate, polyglyceryl dimerate isostearate, polyglyceryl polyricinoleate (Admul WOL 1403), polyglyceryl-1 dipolyhydroxystearate (Dehymuls PGPH), polyglyceryl-2-laurate, polyglyceryl-2 sesquiisostearate, polyglyceryl-3 beeswax (Cera Bellina), polyglyceryl-3 cetyl ether (Chimexane NL), polyglyceryl-3 distearate (Cremophor GS 32), polyglyceryl-3 oleate, polyglyceryl-3 methyl glycose distearate, polyglyceryl-4-caprate (polyglycerol caprate T2010190), polyglyceryl-4 diisostearate / polyhydroxystearate I sebacate (Isolan GPS), and polyglyceryl-4 isostearate.

In yet another embodiment, the oil-in-water emulsion has a dynamic viscosity of more than 2500 mPa· s at 20 °C, preferably a dynamic viscosity of 4500 to 14000 mPa· s at 20 °C. The dynamic viscosity can be measured according to ISO 2555:2018 using a Brookfield DVII+ viscometer.

In an embodiment, the oil-in-water emulsion comprises the oil phase in an amount of 0.1 to 20.0 wt.-%, wherein the oil phase comprises fatty alcohols, alkylcarbonates, alkylesters, waxes, natural oils, or mixtures thereof.

The term "fatty alcohols" encompasses C₆-C₂₈ alcohol, preferably C₁₂-C₂₀ alcohols, wherein the prefix "Cₓ-C_{y}" denotes the number of carbon atoms. The alcohol may be a linear or branched alcohol. Non-limiting examples include lauryl alcohol, myristyl alcohol, cetyl alcohol, stearyl alcohol, and ceryl alcohol. It is in particular preferred that the fatty alcohol is a mixture of C₁₆ and C₁₈ fatty alcohol called cetearyl alcohol.

The term "alkylcarbonate" refers to difunctionalized carbonate alkyl esters. It is preferred that the alkyl groups are identical, but the alkyl groups can be selected independently. The alkyl group may be any C₃-C₂₀ branched, linear or cyclic alkyl. It is preferred that the alkyl group is a C₃-C₂₀ linear alkyl group, even more preferably a capryl group.

The term "wax" refers to natural waxes, such as jojoba wax, and synthetic waxes such as polyglycerin-3.

Non-limiting examples of natural oils comprise canola oil, soy oil, sunflower oil, almond oil, grape oil, avocado oil, castor oil, glycine, jojoba oil, arnica oil, hibiscus oil, licorice oil and oils from *Areca catechu, Crocus sativus, Curcuma longa, Glycyrrhiza glabra,* green tea, *Crataevea murula, Rosmarinus Officinalis,* buckwheat seeds, *Emblica officinale, Ginkgo biloba, Centella asiatica, Psoraliea corylifolia, Citrus limonum, Aloe Vera, matricaria recutita, Thea viridis, Vitis vinifera, Daucus carota, Lycopersicon esculentum, Allium sativum and Hamamelis virginiana.* The natural oil may have various useful properties and can i.a. be used as anti-inflammatory, anti-irritant, antimicrobial or fragrance component.

The oil-in-water emulsion according to the current invention can include further excipients which are known to the skilled person. These excipients are preferably cosmetically, pharmaceutically and/or dermatologically acceptable compounds. For instance, the further excipients are selected from the group of pH-modifiers, buffers, detergents, solubilizers, humectants, fillers, bioadhesives, emollients, preservatives, bactericides, surfactants, perfumes, thickeners (also referred to as thickening agents hereinafter), softening agents, moisturizing agents, oils, fats, polyols, and polymers. Suitable excipients are described in "Cosmetic Formulation of Skin Care Products", Draelos et al., Taylor & Francis Group, 2006.

In an embodiment, the oil-in-water emulsion further comprises a thickening agent, wherein the thickening agent is preferably selected from xanthan gum, polyacrylic acid, cellulose ethers, and mixtures thereof. It is preferred that thickening agent is present in an amount of 0.01 to 5 wt.-%, 0.05 to 3 wt.-% or 0.1 to 0.8 wt.-%. Non-limiting examples of the above cellulose ethers contain methylcellulose, hydroxypropyl cellulose, hydroxyethyl cellulose, and hydroxyethylmethyl cellulose.

In an embodiment, the oil-in-water emulsion further comprises 0.01 to 5.0 wt.-% of an emollient, wherein the emollient is preferably dimethicone and/or cyclopentasiloxane. Emollients are beneficial for skin lubricity and are soothing. In some embodiments, the oil-in-water emulsion comprises humectant agents, such as glycerin, hyaluronic acid, ammonium lactate, and panthenol.

In an embodiment, the weight ratio (b)/(a) is from 2.0 to 15.0, preferably from 3 to 10. This weight ratio ensures a stable emulsion in the presence of ethanol that does not separate into distinct phases.

In an embodiment, the oil-in-water emulsion further comprises a vitamin. Non-limiting examples of vitamins include niacin, biotin, vitamin E (also referred to as tocopherol), vitamin A, vitamin C, and dexpanthenol. Vitamins are antioxidants and can have various additional useful properties for maintaining a healthy skin barrier.

In an embodiment, the oil-in-water emulsion comprises 40.0 to 80.0 wt.-% water phase, 0.1 to 20 wt.-% oil phase, 15.0 to 30.0 wt.-% ethanol, 1.0 to 15.0 wt.-% of an emulsifier, 1.0 to 10.0 wt.-% of cetearyl ethylhexanoate, and 0.01 to 3 wt.-% of a thickening agent.

In a preferred embodiment, the oil-in-water emulsion comprises 40.0 to 80.0 wt.-% water phase, 0.1 to 20 wt.-% oil phase, 15.0 to 30.0 wt.-% ethanol,1.0 to 15.0 wt.-% of an emulsifier, 1.0 to 10.0 wt.-% of cetearyl ethylhexanoate, 0.01 to 3 wt.-% of a thickening agent, 0.01 to 2.0 wt.-% of an emollient, and 0.01 to 1.0 wt.-% of a vitamin.

In an embodiment, the invention relates to the above oil-in-water emulsion for use in the prevention of a bacterial, mycotic, or enveloped viral disease.

In an embodiment, the oil-in-water emulsion is topically applied to the skin, preferably to the hand. In a preferred embodiment, the oil-in-water emulsion is applied according to DIN EN 1500:2017 for at least 10 seconds, at least 30 seconds, at least 60 seconds, or at least 120 seconds.

In an embodiment, the disease is an infection with an enveloped virus belonging to the group consisting *of Poxviridae, Paramyxoviridae, Filoviridae, Orthomyxoviridae, Astroviridae, and Coronaviridae,* or wherein the disease is an infection with a bacterium belonging to the group of *Staphylococcus aureus, Enterococcus hirae, Pseudomonas aeruginosa, Escherichia coli, Haemophilus influenza, and Streptococcus pneumoniae.* It is particularly preferred that the disease is an infection with an influenza virus. It is also particularly preferred that the disease is a bacterial infection with *Staphylococcus aureus, Enterococcus hirae, Escherichia coli* or *Pseudomonas aeruginosa.*

In an embodiment, the disease is an infection with a virus belonging to the family of *Coronaviridae,* preferably an infection with SARS-CoV-2.

### Examples

### Example 1: Skin Tolerability

An oil-in-water emulsion containing 62.375 wt.-% water, 20.00 wt.-% ethanol, 6.00 wt.-% cetearyl ethylhexanoate, 2.50 wt.-% polyglyceryl-3 methylglucose distearate, 2.00 wt.-% glyceryl stearate, 2.00 wt.-% dicapryl carbonate, 2.00 wt.-% panthenol, and 1.50 wt.-% cetearyl alcohol was provided.

A pre-determined amount of 2 mL of said oil-in-water emulsion comprising was topically applied according to DIN EN 1500:2017 to the hand of ten test subjects three times a day over the course of 14 days. The skin tolerability was subsequently measured, wherein
"x" denotes no change in the skin properties of the test subjects;
"n" denotes the occurrence of mild rashes, irritations or dryness; and
"o" denotes the occurrence of a more moist and smoother skin.

After 14 days, all test subjects denoted "o" or "x" and none of the test subjects denoted "n".

### Example 2: In vitro anti-bacterial efficacy

The oil-in-water emulsion of Example 1 was tested undiluted in accordance with DIN EN ISO 1276 against S. *aureus, E. coli, Ec. hirae, P. aeruginosa.* The exposure time was 60 seconds. It was found that the oil-in-water emulsion had substantial anti-bacterial efficacy.

### Example 3: In vitro anti-mycotic efficacy

The oil-in-water emulsion of Example 1 was tested undiluted in accordance with DIN EN ISO 1650 against *C*. *albicans.* The exposure time was 60 seconds It was found that the oil-in-water emulsion had substantial anti-bacterial efficacy.

### Example 4: In vitro antiviral efficacy

The oil-in-water emulsion of Example 1 was tested undiluted in accordance with DIN EN ISO 16777 against Vacciniavirus. The exposure time was 60 seconds. It was found that the oil-in-water emulsion had substantial antiviral efficacy.

## Claims

1. An oil-in-water emulsion comprising
(a) an oil phase;
(b) an aqueous phase; and
(c) an emulsifier,
wherein the emulsion further comprises 15.0 to 30.0 wt.-% ethanol, further comprising 1.0 to 10.0 wt.-% of cetearyl ethylhexanoate as a skin care component.

2. The oil-in-water emulsion according to claim 1, comprising the emulsifier in an amount of 1.0 to 15.0 wt.-%.

3. The oil-in-water emulsion according to claim 1 or 2, wherein the emulsifier is selected from the group of fatty alcohol esters, glycerol fatty acid esters, polyoxyethylene ethers of one or more fatty alcohols, polyglycerol ethers of fatty alcohols, polyglycerol esters of fatty acid, and mixtures thereof.

4. The oil-in-water emulsion according to any one of claims 1 to 3, comprising the oil phase in an amount of 0.1 to 20.0 wt.-%, wherein the oil phase comprises fatty alcohols, alkylcarbonates, alkylesters, waxes, natural oils, or mixtures thereof.

5. The oil-in-water emulsion according to any one of claims 1 to 4, further comprising a thickening agent, wherein the thickening agent is preferably selected from xanthan gum, polyacrylic acid, cellulose ethers, and mixtures thereof.

6. The oil-in-water emulsion according to any one of claims 1 to 5, further comprising 0.01 to 5.0 wt.-% of an emollient, wherein the emollient is preferably dimethicone.

7. The oil-in-water emulsion according to any one of claims 1 to 6, wherein the weight ratio (b)/(a) is from 2.0 to 15.0, preferably from 3 to 10.

8. The oil-in-water emulsion according to any one of claims 1 to 7, further comprising a vitamin.

9. The oil-in-water emulsion according to any one of claim 1 to 8, comprising 40.0 to 80.0 wt.-% water phase, 0.1 to 20 wt.-% oil phase, 1.0 to 15.0 wt.-% of an emulsifier, 1.0 to 10.0 wt.-% of cetearyl ethylhexanoate, and 0.01 to 3 wt.-% of a thickening agent.

10. The oil-in-water emulsion according to any one of claims 1 to 9 for use in the prevention of a bacterial, mycotic, or enveloped viral disease.

11. The oil-in-water emulsion for use according to claim 10, wherein the oil-in-water emulsion is topically applied to the skin, preferably to the hand.

12. The oil-in water emulsion for use according to claim 10 or 11, wherein the disease is an infection with an enveloped virus belonging to the group consisting *of Poxviridae, Paramyxoviridae, Filoviridae, Orthomyxoviridae, Astroviridae, and Coronaviridae,* or wherein the disease is an infection with a bacterium belonging to the group of *Staphylococcus aureus, Enterococcus hirae, Pseudomonas aeruginosa, Escherichia coli, Haemophilus influenza, and Streptococcus pneumoniae.*

13. The oil-in-water emulsion for use according to any one of claims 10 to 12 wherein the disease is an infection with a virus belonging to the family of *Coronaviridae,* preferably an infection with Sars-CoV-2.

## Patentansprüche

1. Öl-in-Wasser-Emulsion, umfassend
(a) eine Ölphase,
(b) eine wässrige Phase; und
(c) einen Emulgator,
wobei die Emulsion ferner 15,0 bis 30,0 Gew.-% Ethanol umfasst, ferner umfassend 1,0 bis 10,0 Gew.-% Cetearylethylhexanoat als Hautpflegekomponente.

2. Öl-in-Wasser-Emulsion nach Anspruch 1, umfassend den Emulgator in einer Menge von 1,0 bis 15,0 Gew.-%.

3. Öl-in-Wasser-Emulsion nach Anspruch 1 oder 2, wobei der Emulgator aus der Gruppe der Fettalkoholester, der Glycerinfettsäureester, der Polyoxyethylenether eines oder mehrerer Fettalkohole, der Polyglycerinether von Fettalkoholen, der Polyglycerinester von Fettsäuren und deren Mischungen ausgewählt ist.

4. Öl-in-Wasser-Emulsion nach einem der Ansprüche 1 bis 3, umfassend die Ölphase in einer Menge von 0,1 bis 20,0 Gew.-%, wobei die Ölphase Fettalkohole, Alkylcarbonate, Alkylester, Wachse, natürliche Öle oder Mischungen davon umfasst.

5. Öl-in-Wasser-Emulsion nach einem der Ansprüche 1 bis 4, ferner umfassend ein Verdickungsmittel, wobei das Verdickungsmittel vorzugsweise ausgewählt ist aus Xanthan, Polyacrylsäure, Celluloseethern und Mischungen davon.

6. Öl-in-Wasser-Emulsion nach einem der Ansprüche 1 bis 5, ferner umfassend 0,01 bis 5,0 Gew.-% eines Weichmachers, wobei der Weichmacher vorzugsweise Dimethicon ist.

7. Öl-in-Wasser-Emulsion nach einem der Ansprüche 1 bis 6, wobei das Gewichtsverhältnis (b)/(a) von 2,0 bis 15,0, vorzugsweise von 3 bis 10, beträgt.

8. Öl-in-Wasser-Emulsion nach einem der Ansprüche 1 bis 7, ferner umfassend ein Vitamin.

9. Öl-in-Wasser-Emulsion nach einem der Ansprüche 1 bis 8, umfassend 40,0 bis 80,0 Gew.-% Wasserphase, 0,1 bis 20 Gew.-% Ölphase, 1,0 bis 15,0 Gew.-% eines Emulgators, 1,0 bis 10,0 Gew.-% Cetearylethylhexanoat, und 0,01 bis 3 Gew.-% eines Verdickungsmittels.

10. Öl-in-Wasser-Emulsion nach einem der Ansprüche 1 bis 9 zur Verwendung bei der Prävention einer bakteriellen, mykotischen oder viralen Erkrankung mit einem behüllten Virus.

11. Öl-in-Wasser-Emulsion zur Verwendung nach Anspruch 10, wobei die Öl-in-Wasser-Emulsion topisch auf die Haut, vorzugsweise auf die Hand, aufgetragen wird.

12. Öl-in-Wasser-Emulsion zur Verwendung nach Anspruch 10 oder 11, wobei die Erkrankung eine Infektion mit einem umhüllten Virus ist, das zu der Gruppe gehört, die aus *Poxviridae, Paramyxoviridae, Filoviridae, Orthomyxoviridae, Astroviridae,* und *Coronaviridae* besteht, oder wobei die Erkrankung eine Infektion mit einem Bakterium ist, das zu der Gruppe aus *Staphylococcus aureus, Enterococcus hirae, Pseudomonas aeruginosa, Escherichia coli, Haemophilus influenza* und *Streptococcus pneumoniae* gehört.

13. Öl-in-Wasser-Emulsion zur Verwendung nach einem der Ansprüche 10 bis 12, wobei die Erkrankung eine Infektion mit einem Virus ist, das zur Familie der *Coronaviridae* gehört, vorzugsweise eine Infektion mit Sars-CoV-2.

## Revendications

1. Émulsion huile dans eau comportant :
(a) une phase huileuse ;
(b) une phase aqueuse ; et
(c) un émulsifiant,
dans laquelle l'émulsion comporte en outre de 15,0 à 30,0 % en poids d'éthanol, comportant en outre 1,0 à 10,0 % en poids d'éthylhexanoate de cétéaryle en tant que composant de soin pour la peau.

2. Émulsion huile dans eau selon la revendication 1, comportant l'émulsifiant en une quantité de 1,0 à 15,0 % en poids.

3. Émulsion huile dans eau selon la revendication 1 ou 2, dans laquelle l'émulsifiant est choisi parmi le groupe constitué d'esters d'alcools gras, d'esters d'acide gras de glycérol, d'éthers de polyoxyéthylène d'un ou plusieurs alcools gras, d'éthers de polyglycérol d'alcools gras, d'esters de polyglycérol d'acide gras, et de mélanges de ceux-ci.

4. Émulsion huile dans eau selon l'une quelconque des revendications 1 à 3, comportant la phase huileuse en une quantité de 0,1 à 20,0 % en poids, dans laquelle la phase huileuse comporte des alcools gras, des carbonates d'alkyle, des esters d'alkyle, des cires, des huiles naturelles ou des mélanges de ceux-ci.

5. Émulsion huile dans eau selon l'une quelconque des revendications 1 à 4, comportant en outre un agent épaississant, dans laquelle l'agent épaississant est de préférence choisi parmi la gomme de xanthane, l'acide polyacrylique, des éthers de cellulose et des mélanges de ceux-ci.

6. Émulsion huile dans eau selon l'une quelconque des revendications 1 à 5, comportant en outre de 0,01 à 5,0 % en poids d'un émollient, dans laquelle l'émollient est de préférence la diméthicone.

7. Émulsion huile dans eau selon l'une quelconque des revendications 1 à 6, dans laquelle le rapport pondéral (b)/(a) est de 2,0 à 15,0, de préférence de 3 à 10.

8. Émulsion huile dans eau selon l'une quelconque des revendications 1 à 7, comportant en outre une vitamine.

9. Émulsion huile dans eau selon l'une quelconque des revendications 1 à 8, comportant de 40,0 à 80,0 % en poids de phase aqueuse, 0,1 à 20 % en poids de phase huileuse, 1,0 à 15,0 % en poids d'un émulsifiant, 1,0 à 10,0 % en poids d'éthylhexanoate de cétéaryle, et 0,01 à 3 % en poids d'un agent épaississant.

10. Émulsion huile dans eau selon l'une quelconque des revendications 1 à 9 pour une utilisation dans la prévention d'une maladie bactérienne, mycotique à virus enveloppé.

11. Émulsion huile dans eau pour une utilisation selon la revendication 10, dans laquelle l'émulsion huile dans eau est appliquée par voie topique sur la peau, de préférence sur la main.

12. Émulsion huile dans eau pour une utilisation selon la revendication 10 ou 11, dans laquelle la maladie est une infection par un virus enveloppé appartenant au groupe constitué de *Poxviridae, Paramyxoviridae, Filoviridae, Orthomyxoviridae, Astroviridae* et *Coronaviridae,* ou dans laquelle la maladie est une infection par une bactérie appartenant au groupe de *Staphylococcus aureus, Enterococcus hirae, Pseudomonas aeruginosa, Escherichia coli, Haemophilus influenza* et *Streptococcus pneumoniae.*

13. Émulsion huile dans eau pour une utilisation selon l'une quelconque des revendications 10 à 12, dans laquelle la maladie est une infection par un virus appartenant à la famille de *Coronaviridae,* de préférence une infection par Sars-CoV-2.
